(19) Europäisches Patentamt
European Patent Office
Office européen des brevets

(11) Publication number: **0 081 351**
**A1**

(12) **EUROPEAN PATENT APPLICATION**

(21) Application number: 82306445.6

(22) Date of filing: 03.12.82

(51) Int. Cl.[3]: **A 01 N 43/70**
A 01 N 43/64, A 01 N 43/40
C 07 D 405/04
//(A01N43/70, 43/40, 43/20),
(A01N43/64, 43/40, 43/20)

(30) Priority: 04.12.81 US 327649

(43) Date of publication of application:
15.06.83 Bulletin 83/24

(84) Designated Contracting States:
AT BE CH DE FR IT LI NL SE

(71) Applicant: THE DOW CHEMICAL COMPANY
Dow Center 2030 Abbott Road Post Office Box 1967
Midland Michigan 48640(US)

(72) Inventor: Smit, Leonard Leslie, Jr.
243 Mount Aire Circle
Clayton California 94517(US)

(72) Inventor: Gerwick, Ben Clifford
3979 Mulberry Drive
Concord California 94519(US)

(74) Representative: Allard, Susan Joyce et al,
BOULT, WADE & TENNANT 27 Furnival street
London EC4A 1PQ(GB)

(54) **Herbicidal triazinyl compositions with an epoxy butane and method of using.**

(57) A novel herbicidal composition comprising a herbicidal triazine and a substituted 1,1,1-trichloro-3,4-epoxy butane and optionally crop oil. The composition exhibits more than additive herbicidal effect on grassy and broadleaf weeds safely in the presence of corn and sorghum when applied postemergently.

EP 0 081 351 A1

Croydon Printing Company Ltd

# HERBICIDAL TRIAZINYL COMPOSITIONS WITH AN EPOXY BUTANE AND METHOD OF USING

The well known herbicidal triazines have good activity against broadleaf.weeds but at lower rates are frequently not sufficiently active in the control of certain grassy weeds. On the other hand, the epoxy butanes or oxiranes having phenyl or substituted phenyl, or substituted 4-pyridinyl, in the 3-position of the butane chain, such as 1,1,1-trichloro-3,4-epoxy-3--(3,5-dichlorophenyl)butane, 1,1,1-trichloro-3,4--epoxy-3-(3,5-dibromophenyl)butane or 2,6-dichloro--4-(2-(2,2,2-trichloroethyl)oxiranyl)pyridine, are active against many troublesome grassy weeds and are generally tolerated by corn and sorghum, but do not control broadleaf weeds as well as would be desired.

The invention relates to herbicidal compositions containing admixtures of any one or more of the herbicidal triazines with certain epoxy butane compounds, also referred to herein as oxirane compounds and subsequently further described, the admixtures optionally also containing crop oil or crop oil concentrate, and methods of using such compositions to control noxious

weeds particularly in the presence of valuable crops such as corn and sorghum and particularly in postemergence application to corn crops.

The numerous herbicidal symmetric triazines, such as atrazine, and the asymmetric triazines, such as metribuzin, are well known and their use and properties as selective herbicides described in numerous publications.

The oxirane, or epoxy butane, compounds referred to above having phenyl or substituted phenyl on the butane chain, their synthesis and use as herbicides are described in U.S. Patent Nos. 4,211,549, 4,018,801 and 3,719,465 and Canadian Patent 527,462.

The phenyl substituted oxirane compounds are prepared by epoxidizing the requisite substituted styrene of the formula

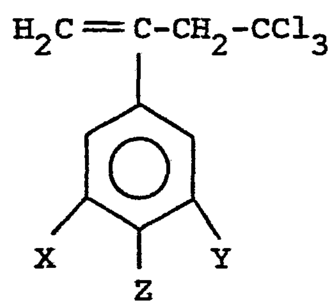

wherein X, Y and Z may each be independently H, or Cl, Br, F, $CF_3$, $NO_2$, alkyl of 1 to 3 carbons or alkoxy of 1 to 3 carbons, with a suitable percarboxylic acid reactant, e.g., peracetic acid or perbenzoic acid.

The pyridinyl substituted oxirane compounds are prepared, e.g., by the Grignard reaction of a lower alkyl carboxylate of the appropriately substituted 4-pyridinyl group, for example, methyl 2,6-dichloro-4-pyridinecarboxylate, with methylmagnesium iodide, followed by acidification to yield the corresponding α,α-dimethyl-4-pyridinemethanol. Treatment of the methanol derivative with $P_2O_5$ removes water and forms the 4-isopropenylpyridine compound, which may then be reacted with $BrCCl_3$ in the presence of a cuprous chloride catalyst to give a 4-(1-bromo-3,3,3-trichloro-1-methylpropyl)pyridine compound which is then dehydrobrominated, e.g., by heating in the presence of a catalyst such as antimony pentachloride, aluminum chloride or a suitable base in an inert organic solvent to provide the corresponding 4-(3,3,3-trichloro-1-methylenepropyl)-pyridine compound. This latter is epoxidized with a peracid such as perbenzoic acid or peracetic acid, preferably employed in a solution buffered with sodium acetate or sodium benzoate, to produce a compound of the following formula

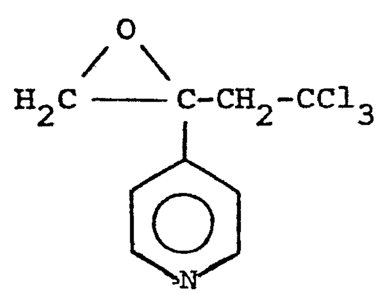

wherein $R_n$ is, independently, bromo, chloro, fluoro, $C_{1-4}$ alkyl, $C_{1-4}$ alkoxy, trifluoromethyl or nitro, and n is 1 or 2.

The symmetric triazines are synthesized by trimerizing cyanuric chloride and replacing one or more of the three chlorines on the ring with, e.g., alkylamino, methoxy or alkylthio groups, by an alkylation type reaction.

The asymmetric triazine metribuzin can be made by reacting tertiary butyl pyruvic acid with the semicarbazone of thiophosgene, viz.,

$$NH_2NH\overset{S}{\overset{\|}{C}}NHNH_2$$

in aqueous reaction medium at reflux temperature whereupon cyclization takes place. Subsequently the sulfur attached to a ring carbon is alkylated by base catalyzed reaction with, e.g., methyl chloride.

It has now been discovered that upon applying to weedy plants early postemergence a combination of one or more herbicidal triazines and a phenyl- or 4-pyridinyl-substituted oxirane, ordinarily admixed with a carrier and optionally with crop oil or crop oil concentrate, and in a range of application rates and proportions more fully set forth hereinafter, there is obtained a greater than predicted herbicidal effect upon many weedy plants while valuable grassy crops such as corn or sorghum, if contacted along with the weedy plants, are undamaged or affected no more than would result from the use of the triazine alone at the dosage employed in the combination.

The new herbicidal compositions of the present invention comprise an agriculturally acceptable carrier and a herbicidally effective amount of a

mixture of one or more herbicidal triazines and a phenyl- or 4-pyridinyl-substituted oxirane, the phenyl being optionally, and the pyridinyl essentially, substituted, the proportions being in the range of, by weight, from 1 to 8 parts, preferably 1 to 4 parts, and more preferably 2 to 3 parts of the herbicidal triazine or triazine admixture per part of oxirane or oxirane admixture, and, up to 2 parts of crop oil per part of triazine component.

In all such uses the present compositions are advantageously employed in an amount sufficient to provide at least 0.28 kgs/ha and preferably at least 0.56 kg/ha of oxirane compound(s).

The composition conveniently adapts itself to the treatment of plant parts postemergently, in valuable field crops such as corn or sorghum, and in the selective control of a good spectrum of both grassy weeds and broadleaf weeds in the presence of such crops. While the oxirane compounds are not all completely non-harmful to crops, such as corn or sorghum, those oxirane compounds and triazines which are somewhat harmful to grassy crops and some broadleaf crops can frequently be employed in the present composition and the composition applied to control weeds in grassy crops or in cotton, pineapple or other plantation crops using a conventional directed spray method of application in which the crop is minimally exposed to the composition spray droplets.

Among compounds of this invention, while phenyl oxiranes substituted at ring positions 3 and 5 and 4-pyridinyl oxiranes substituted at ring positions 2 and 6 with Cl, Br, and/or $CF_3$, generally show good to

excellent selectivity for crops such as corn and sorghum at operable dosages at which good weed control is obtained, unsubstituted phenyl and monosubstituted phenyl and 4-pyridinyl oxiranes, and methyl substituted phenyl and 4-pyridinyl oxiranes, are somewhat less well tolerated. Also, s-triazines having an alkylthio, i.e. -S-alkyl, group on the ring are less well tolerated by such crops than those having a chloro group.

It has now been found that the present composition has the particular advantage that the oxirane component not only contributes its herbicidal characteristics to the composition but also increases the activity of the triazine component, resulting in greater than additive weed control. It is not desired to be bound by any particular theory, but the mode of action is believed to be, and physiological studies indicate, that the oxirane compounds utilized according to the invention inhibit the activity of the plant enzyme glutathione-S-transferase. This enzyme is present at various levels in many triazine tolerant plants, particularly weedy grasses and valuable crops such as corn and sorghum. This enzyme will detoxify triazine herbicides such as chloro-triazines and thio-alkyl-triazines through enzyme dependent conjugation with the tripeptide glutathione. This enzyme dependent conjugation appears to be a primary factor responsible for plant tolerance to the triazine herbicides, e.g., corn tolerance to atrazine. By slowing or inhibiting for a time the above-described detoxification of a triazine or triazine blend with an oxirane compound, the triazine component is retained in a herbicidally effective form and amount within plant tissues for a longer period of time resulting in enhanced activity and increased effectiveness.

Crops tolerant to the oxiranes and triazines in admixture are believed to have capacity to detoxify the oxirane component and/or sufficient triazine detoxification capability, e.g., glutathione-S-transferase activity, to overcome the inhibitory effect. A similar plant response is believed to take place in the slightly larger and more mature grassy and broadleaf weeds which are controlled by the present composition when applied at an earlier stage.

Thus, the present composition is found to be more effective in weed control when applied post-emergently about two to four weeks, and preferably two to three weeks, after seeding the field crop, while the enhancement of the triazine activity diminishes considerably when the weeds reach the seven to eight leaf stage, generally four or more weeks after the crop is planted. Preferably application is made when weedy plants are between the two and six leaf stages.

In order to achieve the desired enhancement of the triazine component or blend used, it is essential to use sufficient oxirane. While the dosage required for optimum enhancement is affected by the particular choice of oxirane and triazine employed, and the tolerance of the particular plant species to be controlled, the compositions are normally advantageously employed in applications in which the oxirane dosage is supplied at a dosage of at least 0.28 kg/ha and preferably 0.56 kg/ha with the triazine component at dosage ratios stipulated hereinabove.

The composition comprising the present mixture of herbicides is sufficiently increased in effectiveness so as to permit the effective utilization of reduced amounts of each of the active components while achieving good broad spectrum weed control of both grassy weeds and broadleaf weeds with little or no damage to the tolerant crops, such as corn or sorghum, or damage from normally less selective triazines or oxiranes may be avoided by employing a directed spray application procedure, minimizing exposure of the crop plants. The crop yields obtained and the control achieved of weeds on the crop land are generally much superior to that obtained when either of the active constituents of the mixture is employed alone.

The terms triazine component and oxirane or epoxy butane component, respectively, refer to a single triazine or blend of two or more triazines, on the one hand, and a single oxirane or epoxy butane of the class described, on the other hand.

The herbicidal triazines employed in the composition and method of the present invention are selected both from the herbicidal asymmetric triazines and the so-called symmetrical triazines.

The suitable symmetrical triazines are selected from the herbicidal triazines of the formula:

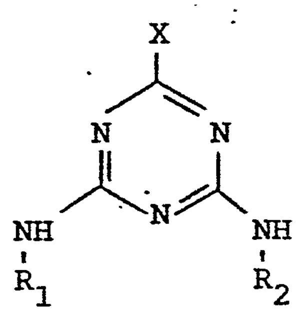

wherein

X is Cl, $SCH_3$ or $SC_2H_5$ and

$R_1$ and $R_2$ are each independently selected from methyl, ethyl, n-propyl, isopropyl, cyclopropyl, 2-cyano-2-methylethyl, sec-butyl, tert-butyl and propoxymethyl.

Suitable asymmetric triazines are particularly to be selected from 4-amino-3-alkylthio-as-triazin-5(4H)-ones with substituents in the 6 position selected from alkyl, haloalkyl, halo, nitro or alkoxy as shown in the following formula

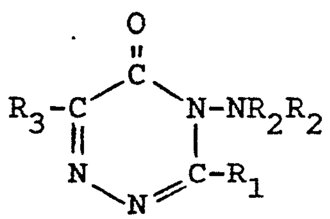

wherein:

$R_1$ is lower alkylmercapto of 1 to 4 carbons, each $R_2$ is independently hydrogen or loweralkyl of 1 to 3 carbons; and

$R_3$ is lower alkyl of 1 to 4 carbons, haloalkyl of 1 to 4 carbons, halo, nitro or alkoxy of 1 to 4 carbons.

The compound metribuzin wherein $R_1$ is $-SCH_3$, both of $R_2$ are hydrogen and $R_3$ is tertiary butyl is most preferred among the asymmetric triazines.

Specific examples of herbicidal triazines suitable for use according to the present invention are: ametryn, atrazine, cyanazine, cyprazine, desmetryn, dipropetryn, prometryn, propazine, simazine, symetryne, terbutryn and metribuzin. Preferred triazines used in

the composition and method of the invention are ametryn, atrazine, cyanazine, propazine, simazine, symetryne and metribuzin. The most preferred triazines are atrazine and cyanazine or a blend or co-mixture thereof.

The oxirane compounds carrying a phenyl or substituted phenyl group used in the present compositions are each yellow oils, freely soluble in most organic solvents, and the oxirane compounds carrying a substituted 4-pyridinyl group are solids melting below 100°C, while the triazines are white crystalline solids, usually fairly high melting and freely soluble in polar organic solvents such as xylene, acetone, methanol or dimethylformamide, but rather low to sparingly soluble in water or aliphatic hydrocarbons such as pentane.

The triazines, accordingly, are best formulated as a flowable concentrate, a wettable powder, or a water dispersible granule, whether formulated alone or in combination with the substituted oxirane compounds. The oxirane compounds may be separately formulated, e.g., as an emulsifiable concentrate and the active ingredients separately dispersed in water and then combined in suitable proportions hereinafter described to form a tank mix, or the respective concentrates may be used to make up a single tank mix directly.

On the other hand, it is convenient to combine both active ingredients together in a concentrate in the form of a suspension-emulsion concentrate, a wettable powder, or a dispersible granule having the active ingredients in desired relative proportions. Such combination concentrate is dispersed in water to form a tank mix ready for field use.

While the triazines have been most generally applied preemergently for weed control in croplands, it is known to utilize crop oil in preparing tank mixes of the triazines for postemergent applications. The crop oils are phytobland, i.e., non-phytotoxic, oils which facilitate penetration by the triazines of the leaf structure of plants but are not harmful to field crops such as corn or sorghum when used at conventional rates. Generally, according to established practices, 0.2 to 2.0 parts of crop oil have been employed per part of triazine used alone. Similar proportions of crop oil may be used in the tank mixes of the present composition, based on the amount of triazine present, but is not believed to be entirely necessary to the obtaining of leaf penetration by the present mixture of active ingredients.

The optional crop oil component of the tank mix composition of the present invention is a petroleum distillate containing primarily paraffinic and naphthenic hydrocarbons and generally containing less than 25 percent by weight aromatics. Such crop oils are known compounds which are disclosed in U.S. Patent 3,997,322. Crop oils are non-phytotoxic and generally have the following range of properties:

| | |
|---|---|
| Gravity, °API/156°C | 31.0-36.0 |
| Viscosity, SUS/37.8°C | 60-120 |
| Viscosity, SUS/98.9°C | 34-38 |
| Flash point, °C | 148.9-204.4 |
| Fire point, °C | 190.6-204.4 |
| Pour temperature, °C | -23 to -6.7 |
| Unsulfonated residue, wt. percent (ASTM) | 75.0-99.9 |
| Refractive index, 25°C | 1.4660-1.4690 |
| Gel Aromatics, wt. percent, max. | 25.0 |
| Distillation range at 10 mm. Hg (ASTM D-1160), °C | 148.9-260 |

Other crop oils are mainly aliphatic petroleum liquids having an average of from seven to nine carbon atoms per molecule, and a boiling temperature in the range of 95° to 150°C. Crop oils of vegetable or animal origin are also sometimes used.

The term "crop oil" when used herein is meant to encompass crop oil concentrates. A crop oil concentrate is a crop oil which contains a greater proportion of surfactants or emulsifiers than a "crop oil". A crop oil concentrate typically contains surfactants or emulsifiers in amounts of up to 30 percent by weight while a crop oil generally contains less than 5 percent by weight of surfactants or emulsifiers.

Crop oils are well known tank mix additives and are commercially available. Examples of suitable crop oils employed in the practice of the present invention, including crop oils, crop oil concentrates and blends of crop oils with an emulsifying agent are: Sun® 11-E; Atplus® 411-F; Booster® Plus E; Agri-Oil® Plus; Agri Dex®; Agrodex®; Herb-Oil® Plus; U.S.S. Spray Adjuvant®; Agicide Activator®; Prime Oil®; Crop Surf® Spray Oil; Adjucide®; Mor-Act®; Sunoco® Superior Spray Oils; Orchex® 696; Paramid® 100; Sun® Superior Spray Oils; and a vegetable oil, such as Bio-Veg.

The present herbicidal compositions in concentrate form are necessarily in the form of one of an emulsion, a suspension, a dispersible granule or a wettable powder, according to intended use. All application forms must contain the active components in fine distribution, i.e., either in an emulsion or finely suspended in a liquid carrier or finely distributed in a wettable powder form carrier or a water dispersible granule.

If it should be desired to employ the present mixed active components for total destruction of vegetative growth, as in fallow land, the herbicidal effect of the components can be increased by the use of carriers which are *per se* phytotoxic, e.g., high boiling mineral oils or chlorohydrocarbons. Mineral oil fractions such as kerosene or diesel oil or coal tar oil may be used as solvents for solutions which can be sprayed onto plants for eradication of plant growth. The active components are added to such oils or liquids directly or after first dissolving in a suitable auxiliary solvent such as xylene.

However, for the selective control of noxious weeds in the presence of the valuable crops referred to herein, it is, at the least, highly desirable to use indifferent carriers which are agriculturally acceptable by virtue of lacking toxicity towards crop plants to be exposed thereto.

For the preferred uses described herein, the active ingredients may be dissolved in an aromatic solvent such as xylene or xylene fraction petroleum distillate, tetrahydronaphthalene or alkylated naphthalene, or an alcohol, such as ethyl alcohol or isopropyl alcohol, a ketone such as acetone or cyclohexanone or a chlorinated hydrocarbon such as tetrachloroethane or ethylene chloride, a glycol ether such as DOWANOL® EB ethylene glycol n-butyl ether, or a polyglycol such as P400 propylene glycol, and combined with one or more suitable emulsifiers for the production of emulsion concentrates which can be diluted with water to form a tank mix in the form of an aqueous emulsion.

Examples of cation active emulsifiers or dispersing agents are quaternary ammonium compounds. Examples of anion active emulsifying agents are soap, soft soap, long chain aliphatic sulfuric acid monoesters, araliphatic sulfonic acids, and long chain alkoxyacetic acids, including, ethoxylated monohydric sulfated alcohol salts of sodium, potassium, alkaline earth metals, ammonium, alkyl amines, alkanolamines or blends thereof (also designated in the art as alkyl polyether alcohol sulfates); ethoxylated sulfated alkylphenol salts of sodium, potassium, alkaline earth metals, ammonium, alkyl amines, or alkanolamines or blends thereof (also designated in the art as alkylaryl polyether alcohol sulfates); and the sodium, potassium, alkaline earth metal, or ammonium salts of alkyl phenoxybenzene disulfonates.

Examples of nonionic emulsifiers are polyglycol ethers of fatty alcohols and polyethylene oxide or polypropylene oxide condensation products and include ethoxylated alkyl phenols (also designated in the art as alkylaryl polyether alcohols); ethoxylated aliphatic alcohols (or alkyl polyether alcohols); ethoxylated fatty acids (or polyoxyethylene fatty acid esters); ethoxylated anhydrosorbitol esters (or polyethylene sorbitan fatty acid esters); ethoxylated polyoxypropylene glycols (polyalkylene oxide block copolymers); ethoxylated polyoxypropylene monohydric alcohols (polyalkylene oxide block copolymers of monohydric alcohols); and ethoxylated polyoxypropylene alkyl phenols (polyalkylene oxide block copolymers of alkyl phenols).

Emulsion concentrates are most usually made up by taking up the triazine and the epoxy butane compound in an emulsifier with or without dispersing

agent added, and if necessary, solvent. The emulsifier and/or dispersing agent are selected as understood in the art to provide a homogeneous concentrate which is capable of being dispersed in water to form, preferably, a stable emulsion in water for use in the field as a tank mix so as to avoid the need for frequent mixing of the tank mix. See the discussion hereinafter regarding hydrophobic-hydrophilic balance values in selecting emulsifiers and blends thereof.

The present herbicidal mixture may also be made up into an emulsifiable concentrate following the procedures and utilizing the emulsifiers and surfactants and solvent aids described in U.S. Patent 3,986,862. The patent describes the preparation of emulsifiable concentrates of metribuzin and alachlor by first taking up the active components and an appropriate emulsifying agent in a small amount of chlorobenzene.

However, since the triazines have such a limited solubility in water, and the solvents which must be used to make up an emulsion concentrate are relatively expensive and the concentrates achievable nonetheless do not contain more than about 10 to 30 percent active ingredient, it is much preferred for other than small scale uses to prepare the present concentrate compositions as (1) a suspension emulsion in which the triazine or triazine blend is suspended in an emulsion of the substituted oxirane compound or compounds, (2) as a wettable powder or (3) as a water dispersible granule product.

Suspension-emulsion type concentrates are made up by grinding the solid triazine component together with one or more suitable emulsifiers and/or dispersing

agent, a clay gellant, and a little solvent in which the triazine is insoluble or very-sparingly soluble. The substituted oxirane compound or compounds may be added before commencing the grinding operation but is preferably added as grinding is being concluded. The emulsifiers and/or dispersing agents are advantageously selected with a hydrophobic-hydrophilic balance value in the range of 7 to 13 and preferably between 10 to 13, as well understood in the art, to provide a stable uniform suspension-emulsion concentrate holding the insoluble triazine component in suspension and the substituted oxirane component emulsified. The concentrate must be capable of holding the triazine in suspension and the substituted oxirane in emulsified form upon dilution to a tank mix for use in the field.

The suspension emulsion of the invention preferably contains ingredients and proportions as follows:

| Ingredient | Weight % |
|---|---|
| substituted oxirane(s) | 12-16 |
| triazine(s) | 32-36 |
| sulfonated naphthalene formaldehyde condensate, such as Daxad® 11 or Morwet® D425 or lignosulfonate salt, such as Polyfon® H or Marosperse® CBO-3 | 0.5-4 |
| clay gellant, such as Bentone® 34 or Attagel® 50 | 0.2-0.8 |
| alkaryl alkoxylate nonionic emulsifier, such as, Triton® X-100, T-Det® N15 or Polyglycol® 26-3 | 2-6 |
| calcium salt or blended amine salts of alkaryl sulfonate anionic emulsifier, such as, Witconate® P1220 or Sponto® AC31-2 | 2-4 |
| solvent: xylene range petroleum distillate, such as, Tenneco® 500-100 or Aromatic® 100 | balance |

Such composition is preferably made up by wet milling the triazine component alone or with most of the additaments and subsequently blending in the substituted oxirane component in admixture with an emulsifier and at least part of the solvent. The suspension emulsion may also be prepared by wet grinding the entire composition.

Wettable powders are prepared by mixing and blending with or without grinding, as may be indicated, the triazine component in finely ground form with a suitable solid such as finely ground talcum, diatomaceous earth, kaolin, bentonite, calcium carbonate, boric acid, tricalcium phosphate, sawdust, powdered cork, charcoal or other dried ground material of vegetable origin. Sometimes it is desirable to first take up the triazine(s) and the substituted oxirane compound(s) in a volatile solvent therefor to assist in the uniform and thorough dispersion throughout the solid carrier. The solvent may be removed and the resulting powder used as a dust. In any event, the triazine(s) should be reduced to an average particle size of less than 20 microns, preferably less than 10 microns and even more preferably in the range of 1 to 5 microns.

More preferably, an emulsifier and/or dispersant is added to the mixture of active components and solid carrier during the mixing or grinding steps to provide a wettable powder capable of dispersion in water to provide a sprayable composition for field use. To provide for improved properties of the present compositions in water-dispersed tank mix form obtained on dilution of a concentrate with water it may be

desirable to incorporate in the concentrate a protective colloid such as a clay gellant, typically a gum. An example of a suitable gum is a xanthan gum having an average molecular weight of at least 200,000.

The wettable powders of the invention preferably contain, by weight, 70 to 80 percent of the combined active ingredients, with the triazine(s) in finely divided form but generally reduced to the fineness described hereinabove by hammer milling or air milling the entire composition or a part thereof before final blending.

In addition, the composition contains from a total of 2 to 10 percent of anionic and nonionic surface active agents or emulsifiers which may also be selected from dispersants. A more preferred range is 2 to 6 percent in the aggregate and most preferred is 4 to 6 percent.

Additional aids such as antifoaming agents or freezing point depressants may be included in minor amounts, if desired, and the balance of approximately 10 to 25 percent of a solid carrier, preferably synthetic precipitated hydrated silica, or, a ball clay, a kaolin clay, a diatomite or a silicate material.

In general, a water dispersible granular product is made by granulating or agglomerating most any wettable powder formulation that is basically suitable for the active ingredients. Agglomeration is carried out in a conventional manner, e.g., conveniently by use of a pan agglomerator. Illustrative water

dispersible granular products are described in U.S. Patent 3,954,439 which formulations are readily adapted to the present combination of herbicidal active ingredients. British Patent 1,583,449 also describes suitable components and techniques for accomplishing agglomeration in the production of a water dispersible granule product.

The various forms of application can be adapted to the intended use in the usual way by the addition of substances which improve the distribution, the adhesive properties to foliar structure, or resistance to rain or resorption. Such substances include fatty acids, resins, wetting agents, glue, casein and algivates. The biological activity can also be modified or broadened by the addition of bactericides, fungicides, compatible complementary plant growth modifiers, as well as fertilizers, or insecticides if desired. The compositions may also be modified, if desired, by the addition of 0.1 to 5 percent by weight of an antifoaming agent and/or a freezing point depressant. Also a minor amount of a bactericide may be added for the protection of the composition per se.

As indicated hereinabove, it is believed the oxirane component of the composition of the invention functions in a special way by inhibiting certain protective mechanisms of susceptible weeds, typically an enzymatic detoxification of the triazine component within the plant tissues. This is particularly evident in susceptible weeds at early growth stages. In addition to inhibiting the detoxification mechanism towards triazines, by interfering with the action at plant enzymes such as glutathione-S-transferase in the case of grassy weeds, the oxirane component also

contributes inherent herbicidal activity by inhibiting or slowing plant growth. The mechanism of action of the present composition with respect to broad leaf weeds has not been delineated, but the end result observed in terms of enhanced activity is substantially the same as with grassy weeds.

The mechanisms for detoxification of the triazines within plant tissues have been described. See the text "Metabolism of Herbicides In Higher Plants", K. K. Hatzios ad D. Penner, Burgess Publishing Company, Minneapolis, Minnesota 1982, pp. 43-49. The inhibition by oxiranes of the detoxification reaction within grassy weeds has only now been observed. While physiological investigations show that the detoxification of triazines may occur at quite low levels of oxirane the surprisingly enhanced effectiveness of the triazine component is often not observable upon gross observations of plant growth if the oxirane component is applied in admixture at rates below about 0.28 kg/ha. This is because other factors affecting growth of weed plants are often of sufficient magnitude to have equal or greater significance. Thus, as a practical matter, it is usually of advantage to use sufficient of the present composition to provide at least 0.28 kg/ha and preferably at least 0.42 kg/ha and most preferably at least 0.56 kg/ha of triazine component to assuredly obtain marked enhancement of the triazine activity while also contributing the herbicidal effect of the oxirane.

These limits could arise in part because of differing rates of metabolism of the oxirane and triazine components within plant tissues. Moreover, the oxiranes are somewhat volatile upon the leaf surfaces.

Measures which would reduce this volatility would reduce the minimum dosages of the oxirane component below the rates set forth above.

Weeds that are not susceptible by virtue of innate characteristics or by virtue of having matured to a stage, such as the 8 to 10 leaf stage, appear to generate protective detoxifying enzyme material in an amount which overcomes the inhibition by the oxirane. This is clearly the case with tolerant crops, e.g., corn or sorghum.

The herbicidal mixture of the present invention, in which the activity of the triazine component is enhanced, exhibits more than an additive effect in the control of grassy weeds, especially yellow foxtail and giant foxtail, i.e., Setaria species, and crabgrass, shattercane, proso millet, Johnson grass and barnyard grass, as well as broadleaf weeds such as cocklebur, velvetleaf, jimsonweed, coffeeweed, teaweed and sicklepod when applied postemergently at a dose in the range of 0.56 to 4.48 kgs per hectare of active ingredients wherein the individual components are present within the critical range of ratios of about one to about eight parts by weight of the triazine component to one part of the substituted oxirane compound, but in which there is provided at least 0.28 kg/ha of oxirane compound. It is more preferred that said ratio is about one to about four parts triazine component per part of substituted oxirane compound, while the most preferred ratio is 2 to 3 parts triazine component per part substituted oxirane compound.

The total amount of active material applied is preferably 1.4 to 3.36 kgs per hectare, and most preferably 1.68 to 2.52 kgs/hectare, preferably of the mixture of any one or more of atrazine or cyanazine with any of 1,1,1-trichloro-3,4-epoxy-3-(3,5-dichlorophenyl)butane; 1,1,1-trichloro-3,4-epoxy-3-(3-chlorophenyl)butane; and 1,1,1-trichloro-3,4-epoxy-3-(2,6--dichloropyridine-4-yl)butane and most preferably with the 3,5-dichlorophenyl substituted epoxy butane. Such combinations are most preferred for controlling weeds in corn and sorghum.

When crop oil is used, it may be employed at the rate of 0.9463 L, i.e., about 2.13 kgs/ha, or approximately 0.2 parts to 2.0 parts crop oil per part of combined active ingredients, but more preferably about one part oil per part of the most preferred triazine-epoxy butane combinations. Wherein the concentrate employed does not contain crop oil, the same may be added during preparation of the tank mix for field use.

In formulating the present herbicidal compositions, it is essential to maintain the relative proportions specified hereinabove and to provide an effective amount of the active components in the finished tank mix composition in order to obtain the desired synergistic effect. The concentration of the active components considered together in liquid formulations employed to supply the desired dosage for field application generally is from 0.00001 to 50 percent by weight although compositions employing as high as a 90 percent concentration may be employed depending

on the type of concentrate and the concentration achievable therein and generally at a concentration of at least 0.01 percent by weight.

In compositions to be employed as concentrates, the mixture of herbicidal components may be present in admixture in a carrier therefor at a concentration of from 5 to 90 percent by weight, although concentrations of suspension emulsions containing significantly more than 50 percent by weight combined active ingredients are not readily prepared, and generally the practical upper limit for most wettable powder formulations of the invention is 80 percent by weight of total active components, while a water dispersible granule product may be made to contain as high as 90 percent by weight active ingredients.

The triazine component may be supplied separately, if desired, as any of a flowable suspension concentrate, a wettable powder, a dispersible granule type formulation or an emulsifiable concentrate, while the substituted oxirane compound may be conveniently supplied as an emulsion concentrate, wettable powder or water dispersible granular material, and the two combined in appropriate amounts in aqueous medium in a tank mix containing the two active components in the range of weight ratios recited hereinabove for field application. An emulsion concentrate wettable powder or water dispersible granule containing the substituted oxirane may be made up into a tank mix together with most any of the commercially supplied concentrates of the herbicidal triazines.

The herbicidal triazines are presently marketed mainly in wettable powder form while some are supplied in the form of a water dispersible granule. These are

prepared basically as described hereinabove for the combination concentrate of the invention, by grinding the components together and granulating in the case of the granular product.

The substituted oxiranes when supplied separately are expected to be in the form of emulsion concentrates rather readily prepared from the technical grade oil or crystalline form of unmodified active ingredient. See the discussion hereinabove of emulsion concentrates.

A suitable emulsion concentrate of substituted oxirane compound contains, by weight, 43 percent of the oxirane compound, 3.25 percent of Sponto® P10-20P alkaryl sulfonate salt 1.75 percent of a blend of Makon® 30 and Makon® 14 alkylphenol ethoxylates and the balance Tenneco® 500-100 solvent and inerts.

As indicated hereinabove, the present herbicidal combination is tolerated well by crops, such as corn and sorghum or certain broadleaf and plantation crops while controlling in an enhanced manner a number of grassy weeds and broadleaf weeds occurring in such crops and also controlling other weeds, albeit in an effective, but not necessarily surprising enhanced manner. The specified weeds are not each controlled in an enhanced manner by each specified herbicide combination throughout the specified application rates broadly specified herein, but substantially throughout the range of rates. The range of effective dosage rates for each combination may readily be determined by those skilled in the art with the present teachings at hand. Accordingly, the present composition is useful throughout the

range of compositions set forth and described herein. At lower ratios of triazine to oxirane compound enhanced activity of the components towards many grassy and broadleaf weeds is exhibited, while at higher ratios, such as about 6:1 to 8:1, enhancemet is exhibited towards many grassy weeds but activity towards many broadleaf weeds, through excellent, tends not to evidence enhancement.

The triazines in general are not at all well tolerated by such crops as sugar beets, tobacco, oats or many vegetable crops and this property seems unaffected by the other components utilized in the present composition, but as indicated above weeds are nonetheless controllable in those crops that are either inherently tolerant or are amenable to directed spray application of the present composition.

The enhanced action referred to herein is most definitely and advantageously exhibited upon making a postemergent application to the foliage of the noxious weeds while in the 2 to 3 or 4 to 6 leaf stage and in the presence of the tolerant crop.

The following examples illustrate the novel herbicidal compositions of the invention and the method of their use.

Example 1

Method A

A wettable powder containing both herbicidal triazine and substituted oxirane is made up as follows:

Atrazine is air milled or hammer milled to less than 20 micron volume or mass median diameter, preferably to less than 10 microns and optimally to a size in the 1 to 5 micron range. The comminuted atrazine is blended throughly with 1,1,1-trichloro-3,4-epoxy-3--(3,5-dichlorophenyl)butane, and with a surfactant and a dispersant, antifoaming additive and a precipitated hydrated silica according to the following composition:

| Components | Weight % |
|---|---|
| 1,1,1-Trichloro-3,4-epoxy-3--(3,5-dichlorophenyl)butane | 20 |
| Atrazine | 60 |
| Morwet® DB (alkyl aryl sulfonate) | 4 |
| Polyfon® H (lignosulfonate) | 2 |
| Dow Corning Antifoam® A (dimethoxysiloxane) | 0.1 |
| Hi Sil® 233 (ppt hydrated silica and inerts) | 13.9 |

Method B

The entire composition set forth immediately above is air milled until the atrazine reaches the requisite fineness.

On dispersing either of the foregoing compositions in water at the desired rates .012 to .06 kg/L, a series of tank mixes is obtained in which the atrazine is uniformly suspended and the substituted oxirane is uniformly dispersed in either emulsified form or in suspended form sorbed on the carrier particles and the tank mixes are readily sprayable in postemergence applications.

Example 2

A wettable powder of atrazine and substituted oxirane is made up in a manner similar to that described in Example 1 but having the following composition:

| Components | Weight % |
|---|---|
| 1,1,1-Trichloro-3,4-epoxy-3--(3,5-dichlorophenyl)butane | 26.6 |
| Atrazine | 53.2 |
| Makon® 10 (alkylphenol ethoxylate) | 3.0 |
| Daxad® #27 (polymerized alkylaryl sulfonate) | 3.0 |
| Hi Sil® 233 | 7.0 |
| Barden® clay (kaolin) and inert | 7.0 |

A portion of the blended material is made up in a series of tank mixes as in Example 1 and the same fine results are observed.

Other portions of the blended wettable powder are pan agglomerated on the addition of, respectively 20, 30 and 40 percent by weight water and carrying out conventional agglomeration techniques and thereafter drying the resulting particles to a moisture content in the range of 1.5 to 3 percent by weight. The resulting products are each water dispersible granular products which form excellent stable, sprayable tank mixes when mixed with water at the same rates as set forth above for the wettable powders of Example 1.

Similar fine results are obtained when the silica and Barden® clay of Examples 1 and 2 are replaced

with other carriers such as the ball and kaolin clays, diatomites and silicates. Both nonionic and anionic surface active agents may be used alternatively in the concentration range of 2 to 10 percent by weight, preferably 2 to 6 percent and most preferably 4 to 6 percent.

Example 3

A suspension-emulsion concentrate is prepared from atrazine and from the dichlorophenyl oxirane, 1,1,1-trichloro-3,4-epoxy-3-(3,5-dichlorophenyl)butane. The atrazine is first wet milled in a xylene range petroleum distillate, Tenneco® 500-100, or in a non-phyto bland crop oil to a fineness in the range described in Example 1 and thereafter the requisite amounts of dichlorophenyl oxirane and surface active materials are added to suspend the atrazine and emulsify the dichlorophenyl oxirane, producing a suspension-emulsion having, in respective runs, the following compositions:

Composition A

| Components | Weight % |
|---|---|
| Dichlorophenyl oxirane | 16 |
| Atrazine | 36 |
| Daxad® 11 sulfonated naphthalene formaldehyde condensate | 4 |
| Bentone® 34 clay gellant | 0.8 |
| Triton® X-100 alkylaryl alkoxylate | 3 |
| Tenneco® 500-100 solvent | balance |

Composition B

| Components | Weight % |
|---|---|
| Dichlorophenyl oxirane | 16 |
| Atrazine | 36 |
| Daxad® 11 sulfonated naphthalene formaldehyde condensate | 1 |
| Polyfon® H lignosulfonate | 2 |
| Bentone® 34 clay gellant | 0.4 |
| Triton® X-100 alkylaryl alkoxylate | 3 |
| Sun Spray® 11 N crop oil | balance |

Composition C

| Components | Weight % |
|---|---|
| Dichlorophenyl oxirane | 16 |
| Atrazine | 36 |
| Bentone® 34 clay gellant | 0.8 |
| Triton® X-100 alkylaryl alkoxylate | 3 |
| Witconate® P-1220 alkylaryl sulfonate calcium salt | 3 |
| Tenneco® 500-100 solvent | balance |

The resulting suspension-emulsion concentrates obtained in each case hold the atrazine smoothly suspended and the dichlorophenyl oxirane emulsified in the concentrate as well as in the tank mix obtained in blending the concentrate with water at the rate of 0.012 kg/L of water and the tank mix is readily sprayable.

Example 4

Method A

0.473 L of an emulsion concentrate containing, by weight, 43 percent of 1,1,1-trichloro-3,4-epoxy-3-(3,5-dichlorophenyl)butane ("dichlorophenyl butane"), 3.2 percent of Sponto® P10-20P calcium dodecylbenzene sulfonic acid, 1.75 percent of nonionic surfactants consisting of nonyl phenol condensed with from 14 to 30 moles of ethylene oxide, about 46 percent petroleum distillate and the balance inerts is thoroughly dispersed in 37.85 L of water.

1.59 Kgs of a commercial flowable suspension concentrate of atrazine triazine herbicide containing, by weight 43 percent of the triazine in addition to surfactants and/or dispersants and solid suspension aids is thoroughly dispersed in 37.85 L of water.

37.85 L of the dispersed dichlorophenyl butane and 37.85 L of the suspended triazine are thoroughly mixed together to provide a tank mix containing, by weight, 0.3 percent of the dichlorophenyl butane and 0.9 percent of the triazine. On applying 187 L/ha to crop land there is provided 0.56 kgs/ha of the dichlorophenyl butane compound and 1.68 kgs/ha of the triazine.

Method B

The atrazine flowable suspension concentrate and the emulsion concentrate of dichlorophenyl oxirane are thoroughly mixed together initially in 26.5 to 37.85 L of water and the balance of 75.7 L of water, i.e., 49.2 to 37.85 L of additional water is combined

with the initial aqueous dispersion to form a tank mix with the same characteristics as described using Method A.

Example 5

In a series of tests carried out to demonstrate the greater than additive effect obtained on applying combinations of 1,1,1-trichloro-3,4-epoxy-3-(3,5-dichlorophenyl)butane with the triazines, atrazine and cyanazine, respectively, in the control of growing yellow foxtail, giant foxtail and crabgrass, each in the 3 to 4 leaf stage, the epoxy butane was provided in the form of an emulsion concentrate containing, by weight, 43 percent of the epoxy butane and 5 percent of surfactant. The emulsion concentrate was dispersed by blending the requisite amount in 0.03 L of water containing 0.05% by volume of a nonionic surfactant containing principally blends of oil-soluble sulfonates with polyoxyethylene ethers which served as additional nonionic spray tank additive.

The atrazine was provided in the form of the commercial product AATREX 4L flowable concentrate containing, by weight, 42 percent of atrazine surfactant plus suspension aids. The requisite amount of flowable concentrate was thoroughly and vigorously mixed with 0.03 L of water containing 0.05% by volume of the nonionic additive described above to provide an aqueous suspension of atrazine.

The cyanazine was provided in the form of BLADEX® 80W wettable powder containing, by weight, 80 percent of cyanazine, surfactant, and the balance

substantially solid excipient carrier. The requisite amount of the wettable powder was vigorously mixed with 0.03 L of water containing 0.05% by volume of the described nonionic additive to provide a sprayable suspension.

In a first series of runs, the requisite amount of dispersed emulsified substituted oxirane compound was admixed with dispersed suspended atrazine to provide tank mixes containing (a) 0.006 kgs/L atrazine and .0015 kgs/L substituted oxirane, (b) 0.006 kgs/L atrazine and 0.002 kgs/L substituted oxirane, (c) 0.006 kgs/L atrazine and 0.003 kgs/L substituted oxirane, and (d) 0.003 kgs/L of each of atrazine and substituted oxirane. Both of the concentrates of individual actives were also diluted separately to contain 0.006 kgs/L active ingredient. All were made up to contain 0.05 percent by volume of the described nonionic surfactant additive.

Yellow foxtail seedlings were grown in pots under outdoor conditions to healthy, vigorous plants in the 3-4 leaf stage. The plants were brought into the greenhouse and the soil shielded with about 0.5 inch vermiculite and the plants treated in respective groups of about 5 plants with 3 replicates of each group. Each of the seven tank mixes described above were applied to respective sets of replicated groups of yellow foxtail plants by spraying thereon at the rate of 187 L/ha and the vermiculite removed immediately. Additional plants were also treated with water containing only the described nonionic surfactant additive which served as a blank. A quantity of each tank mix

was then diluted with an equal volume of water containing 0.05 percent by weight of the described nonionic surfactant additive and respectively sprayed upon respective replicated groups of yellow foxtail plants, and the serial dilution and spraying carried out five additional times or steps. The plants were maintained under good growing conditions in the greenhouse for seven days and then were examined to ascertain the extent of control, i.e., reduction in growth, obtained and scored on the basis of 0 to 100 where 100 represents kill of the plants and 0 represents no reduction in growth.

The results of these runs are summarized in Table 1 wherein the percent control readings are averaged for each set of three replicates. A blank test was run without active ingredient but containing Sponto® 712 surfactant. It gave no control.

TABLE 1

Control of Yellow Foxtail

| Run No. | Triazine Compound | Dosage, kg/ha | Epoxy Butane Compound | Dosage, kg/ha | Ratio of Chemicals A or C to EB-1 | Expected Control In Percent | Actual Control In Percent | Percent Increase Over Expected Control |
|---|---|---|---|---|---|---|---|---|
| 1 | A | 1.12 | - | - | - | - | 95 | - |
| 2 | A | 0.56 | - | - | - | - | 70 | - |
| 3 | A | 0.28 | - | - | - | - | 57 | - |
| 4 | C | 1.12 | - | - | - | - | 82 | - |
| 5 | C | 0.56 | - | - | - | - | 68 | - |
| 6 | C | 0.28 | - | - | - | - | 48 | - |
| 7 | - | - | EB-1 | 1.12 | - | - | 57 | - |
| 8 | - | - | EB-1 | 0.56 | - | - | 52 | - |
| 9 | - | - | EB-1 | 0.28 | - | - | 37 | - |
| 10 | A | 1.12 | EB-1 | 0.28 | 4:1 | 97 | 97 | 0 |
| 11 | C | 1.12 | EB-1 | 0.28 | 4:1 | 89 | 68 | -24 |
| 12 | A | 1.12 | EB-1 | 0.37 | 3:1 | 97[a] | 95 | -2 |
| 13 | C | 1.12 | EB-1 | 0.37 | 3:1 | 89[a] | 97 | 9 |
| 14 | A | 1.12 | EB-1 | 0.56 | 2:1 | 98 | 100 | 2 |
| 15 | A | 0.56 | EB-1 | 0.28 | 2:1 | 81 | 98 | 21 |
| 16 | C | 1.12 | EB-1 | 0.56 | 2:1 | 91 | 97 | 7 |
| 17 | C | 0.56 | EB-1 | 0.28 | 2:1 | 80 | 95 | 19 |
| 18 | A | 0.56 | EB-1 | 0.56 | 1:1 | 86 | 98 | 14 |
| 19 | A | 0.28 | EB-1 | 0.28 | 1:1 | 73 | 83 | 14 |
| 20 | C | 0.56 | EB-1 | 0.56 | 1:1 | 85 | 88 | 4 |
| 21 | C | 0.28 | EB-1 | 0.28 | 1:1 | 67 | 57 | -15 |

TABLE 1 con't

Control of Yellow Foxtail

A blank test run without active ingredient in water containing SPONTO® 712 surfactant gave no control.

A = atrazine; C = cyanazine; EB-1 = substituted oxirane compound, 1,1,1-trichloro-3,4-epoxy-3--(3,5-dichlorophenyl)butane.

$$\text{Expected control} = \text{\% control by A (or B)} + \text{\% control by EB minus } \frac{\text{\% control by A (or B)} \times \text{\% control by EB}}{100}$$

$$\text{Percent increase over expected control} = \frac{100\ \text{\% (actual control)}}{\text{\% expected control}} - 100$$

The results of Table 1 show in a few instances the practical problem of obtaining evidence of enhanced activity when the individual components are used at relatively high rates. This is due to the fact that individual components independently give good control. In this table and following tables negative results were occasionally obtained in the runs reported here although in other instances enhancement was exhibited upon using the same compositions of the invention under different crop conditions.

In a second series of runs utilizing the substituted oxirane compound 1,1,1-trichloro-3,4-epoxy-3-(3,5-dichlorophenyl)butane, crabgrass seedlings germinated and grown to the 3 to 4 leaf stage outside the greenhouse and grouped as were the yellow foxtail seedlings were brought into the greenhouse and respective groups sprayed at the rate of 187 L/ha with the tank mixes containing (a) 0.006 kg/L atrazine and 0.002 kg/L substituted oxirane compound, (b) 0.006 kg/L cyanazine and 0.002 kg/L substituted oxirane compound, (c) 0.006 kg/L atrazine, (d) 0.006 kg/L cyanazine and (e) 0.006 kg/L substituted oxirane compound, and (f) water-surfactant blank. A quantity of each tank mix was then diluted with an equal volume of water containing 0.05 percent by weight of the described nonionic surfactant additive and additional respective groups of crabgrass seedlings were sprayed with the diluted tank mixes. The serial dilution procedure was followed several more times for each tank mix. In each case, vermiculite was used to shield the soil as was described above for the treatment of yellow foxtail seedlings.

The crabgrass seedlings were then kept in the greenhouse under good growing conditions and after 8 days were examined to ascertain the extent of control obtained and scored as described above. The results of these runs are summarized in Table 2 wherein the readings are again averaged for each set of three replicated groups. The blank gave no control.

TABLE 2

Control of Crabgrass

| Run No. | Triazine Compound | Dosage, Per Acre | Epoxy Butane Compound | Dosage, Per Acre | Ratio of Chemicals A or C to EB-1 | Expected Control In Percent | Actual Control In Percent | Percent Increase Over Expected Control |
|---|---|---|---|---|---|---|---|---|
| 1 | A | 1.12 | - | - | - | - | 7 | - |
| 2 | A | 0.56 | - | - | - | - | 0 | - |
| 3 | C | 1.12 | - | - | - | - | 43 | - |
| 4 | C | 0.56 | - | - | - | - | 33 | - |
| 5 | C | 0.28 | - | - | - | - | 17 | - |
| 6 | - | - | EB-1 | 2.24 | - | - | 80 | - |
| 7 | - | - | EB-1 | 1.12 | - | - | 78 | - |
| 8 | - | - | EB-1 | 0.56 | - | - | 75 | - |
| 9 | - | - | EB-1 | 0.28 | - | - | 60 | - |
| 10 | A | 1.12 | EB-1 | 0.37 | 3:1 | 61[a] | 77 | 26 |
| 11 | C | 1.12 | EB-1 | 0.37 | 3:1 | 76[a] | 97 | 28 |

A = atrazine; C = cyanazine; EB-1 = 1,1,1-trichloro-3,4-epoxy-3(3,5-dichlorophenyl)butane.

Expected control and percent increase calculated as in Table 1.

a = Control values for EB at 0.37 kg/ha read off smooth curve drawn of plot made using actual readings shown above.

In a third series of runs, giant foxtail seedlings were germinated, handled and treated in the same manner as described above for crabgrass seedlings.

The results of these runs are summarized in Table 3 wherein the readings are again averaged values for each set of three replicated groups.

TABLE 3

Control of Giant Foxtail

| Run No. | Triazine Compound | Dosage, kg/ha | Epoxy Butane Compound | Dosage, kg/ha | Ratio of Chemicals A or C to EB-1 | Expected Control In Percent | Actual Control In Percent | Percent Increase Over Expected Control |
|---|---|---|---|---|---|---|---|---|
| 1 | A | 1.12 | - | - | - | - | 10 | - |
| 2 | A | 0.56 | - | - | - | - | 0 | - |
| 3 | C | 1.12 | - | - | - | - | 10 | - |
| 4 | C | 0.56 | - | - | - | - | 10 | - |
| 5 | - | - | EB-1 | 2.24 | - | - | 50 | - |
| 6 | - | - | EB-1 | 1.12 | - | - | 27 | - |
| 7 | - | - | EB-1 | 0.56 | - | - | 17 | - |
| 8 | - | - | EB-1 | 0.28 | - | - | 5 | - |
| 9 | A | 1.12 | EB-1 | 0.37 | 3:1 | 13[a] | 75 | 477 |
| 10 | C | 1.12 | EB-1 | 0.37 | 3:1 | 13[a] | 90 | 592 |

A =atrazine; C = cyanazine; EB-1 = 1,1,1-trichloro-3,4-epoxy-3(3,5-dichlorophenyl)butane.

Expected control and percent increase calculated as described in Table 1.

a = Control values for EB at 0.37 kg/ha read off smooth curve drawn of plot of actual readings shown above.

In a fourth series of experiments, velvetleaf (Abutilon theophrasti) was grown from seed to the 4 leaf (approximately 5 cm) stage and treated as described in the previous examples except applications were made in 1% by volume Sun® 11E crop oil dilute emulsion containing 0.1% by volume of a nonionic surfactant containing principally blends of oil-soluble sulfonates with polyoxyethylene ethers. The results of these tests are presented in Table 4 as mean control ratings.

TABLE 4

Control of Velvetleaf

| Run No. | Triazine Compound | Dosage, kg/ha | Epoxy Butane Compound | Dosage, kg/ha | Ratio of Chemicals A or C to EB-1 | 7 Day Evaluation: Expected Control In Percent | 7 Day Evaluation: Actual Control In Percent | 7 Day Evaluation: Percent Increase Over Expected Control |
|---|---|---|---|---|---|---|---|---|
| 1 | A | 2.24 | - | - | - | - | 70 | - |
| 2 | A | 1.12 | - | - | - | - | 50 | - |
| 3 | A | 0.56 | - | - | - | - | 40 | - |
| 4 | A | 0.28 | - | - | - | - | 30 | - |
| 5 | C | 2.24 | - | - | - | - | 65 | - |
| 6 | C | 1.12 | - | - | - | - | 70 | - |
| 7 | C | 0.56 | - | - | - | - | 60 | - |
| 8 | C | 0.28 | - | - | - | - | 35 | - |
| 9 | - | - | EB-1 | 2.24 | - | - | 65 | - |
| 10 | - | - | EB-1 | 1.12 | - | - | 60 | - |
| 11 | - | - | EB-1 | 0.56 | - | - | 45 | - |
| 12 | - | - | EB-1 | 0.28 | - | - | 40 | - |
| 13 | A | 1.12 | EB-1 | 0.56 | 2:1 | 73 | 95 | 30 |
| 14 | A | 0.56 | EB-1 | 0.28 | 2:1 | 64 | 70 | 9 |
| 15 | A | 1.12 | EB-1 | 0.37 | 3:1 | 73[a] | 85 | 16 |

TABLE 4 Con'd

Control of Velvetleaf

| Run No. | Triazine Compound | Dosage, kg/ha | Epoxy Butane Compound | Dosage, kg/ha | Ratio of Chemicals A or C to EB-1 | 7 Day Evaluation: Expected Control In Percent | 7 Day Evaluation: Actual Control In Percent | 7 Day Evaluation: Percent Increase Over Expected Control |
|---|---|---|---|---|---|---|---|---|
| 16 | C | 1.12 | EB-1 | 0.56 | 2:1 | 84 | 95 | 13 |
| 17 | C | 0.56 | EB-1 | 0.28 | 2:1 | 76 | 75 | -1 |
| 18 | C | 1.12 | EB-1 | 0.37 | 3:1 | 84[a] | 77 | -8 |

A = atrazine; C = cyanazine; EB-1 = Same substituted oxirane as in Table 1.

Expected control and percent increase calculated as described in Table 1.

a = Control values for EB-1 at 0.37 lbs/acre read off smooth curve drawn of plot of actual readings shown above.

Utilizing experimental techniques similar to those described previously, a fifth series of experiments were conducted to examine the interaction of epoxy butane compounds with symmetrical triazines other than atrazine and cyanazine. Additional triazines selected for examination were propazine (a 2-Cl symmetrical triazine), simetryne (a 2-$SCH_3$ symmetrical triazines), prometryn (a 2-$SCH_3$ symmetrical triazine), ametryn (a 2-$SCH_3$ symmetrical triazine), and simazine (a 2-Cl symmetrical triazine). Ametryn was used as an 80% wettable powder formulation sold by Ciba-Geigy under the trade name EVIK® 80W. Propazine was used as the 80% wettable powder Milogard® (Ciba-Geigy), symetryne as an 80% wettable powder utilizing talcum powder as the solid inert carrier, and simazine as Princep 80W (Ciba-Geigy) wettable powder.

A serial dilution of each triazine by itself and in combination with an emulsifiable concentrate formulation of the substituted oxirane compound, 1,1,1--trichloro-3,4-epoxy-3-(3,5-dichlorophenyl)butane, was performed utilizing 1.25% Atplus® 411F crop oil concentrate as the dilutent. Velvetleaf, (Abutilon theophrasti), shatter cane (Sorghum bicolor), crabgrass (Digitaria spp.), proso millet (Panicum miliaceum), and yellow foxtail (Setaria lutescens), were used as test species during the course of these trials, and all were grown from seed in a sandy loam soil. Vermiculite was used to shield the soil surface as previously described.

Experimental details unique to a given run are listed as footnotes in Tables 5 through 12, where the results are tabulated for control of the weeds by

the selected triazines, the epoxy butane compound, and the triazine-epoxy-butane combinations. The abreviation EB-1 refers to the substituted epoxy butane compound 1,1,1-trichloro-3,4-epoxy-3-(3,5-dichlorophenyl)butane throughout these tables.

TABLE 5

Control of Proso Millet*

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Ametryne | 1.12 | | | | | 85 | |
| 2 | Ametryne | 0.56 | | | | | 75 | |
| 3 | Ametryne | 0.28 | | | | | 65 | |
| 4 | | | EB-1 | 0.56 | | | 15 | |
| 5 | | | EB-1 | 0.28 | | | 10 | |
| 6 | Ametryne | 1.12 | EB-1 | 0.56 | 2:1 | 87 | 99 | 14 |
| 7 | Ametryne | 0.56 | EB-1 | 0.28 | 2:1 | 78 | 90 | 15 |

*At time of chemical application the proso millet was approximately 12.7 cm tall. Above evaluation was taken 6 days following application.

TABLE 6

Control of Shattercane*

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Ametryne | 1.12 | | | | | 65 | |
| 2 | Ametryne | 0.56 | | | | | 40 | |
| 3 | Ametryne | 0.28 | | | | | 25 | |
| 4 | | | EB-1 | 0.56 | | | 5 | |
| 5 | | | EB-1 | 0.28 | | | 0 | |
| 6 | Ametryne | 1.12 | EB-1 | 0.56 | 2:1 | 67 | 65 | -3 |
| 7 | Ametryne | 0.56 | EB-1 | 0.28 | 2:1 | 40 | 55 | 38 |

*At time of treatment the shattercane was roughly 12.7 cm tall. Above evaluation was taken 6 days after chemical application.

TABLE 7

Control of Velvetleaf*

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Ametryne | 1.12 | | | | | 95 | |
| 2 | Ametryne | 0.56 | | | | | 90 | |
| 3 | Ametryne | 0.28 | | | | | 80 | |
| 4 | | | EB-1 | 0.56 | | | 20 | |
| 5 | | | EB-1 | 0.28 | | | 10 | |
| 6 | Ametryne | 1.12 | EB-1 | 0.56 | 2:1 | 96 | 100 | 4 |
| 7 | Ametryne | 0.56 | EB-1 | 0.28 | 2:1 | 91 | 98 | 8 |

*At time of treatment the velvetleaf was 3.8 to 5 cm tall. Above evaluation was made 6 days after treatment.

TABLE 8

Control of Crabgrass*

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Ametryne | 1.12 | | | | | 85 | |
| 2 | Ametryne | 0.56 | | | | | 65 | |
| 3 | Ametryne | 0.28 | | | | | 45 | |
| 4 | | | EB-1 | 0.56 | | | 20 | |
| 5 | | | EB-1 | 0.28 | | | 10 | |
| 6 | Ametryne | 1.12 | EB-1 | 0.56 | 2:1 | 88 | 99 | 13 |
| 7 | Ametryne | 0.56 | EB-1 | 0.28 | 2:1 | 69 | 95 | 38 |

*Crabgrass was approximately 5 cm tall at time of treatment. Evaluation shown above was made 6 days after treatment.

TABLE 9

Control of Crabgrass*

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Propazine | 1.12 | - | - | - | - | 65 | |
| 2 | Propazine | 0.56 | | | | | 30 | |
| 3 | Propazine | 0.28 | | | | | 5 | |
| 4 | Simetryne | 1.12 | | | | | 75 | |
| 5 | Simetryne | 0.56 | | | | | 30 | |
| 6 | Simetryne | 0.28 | | | | | 5 | |
| 7 | | | EB-1 | 0.56 | | | 30 | |
| 8 | | | EB-1 | 0.28 | | | 15 | |
| 9 | Propazine | 1.12 | EB-1 | 0.56 | 2:1 | 76 | 99 | 31 |
| 10 | Propazine | 0.56 | EB-1 | 0.28 | 2:1 | 41 | 95 | 132 |
| 11 | Simetryne | 1.12 | EB-1 | 0.56 | 2:1 | 83 | 99 | 19 |
| 12 | Simetryne | 0.56 | EB-1 | 0.28 | 2:1 | 41 | 95 | 132 |

*Crabgrass was about 7.6 cm tall at time of treatment. Above evaluation was taken 8 days after treatment.

TABLE 10

Control of Velvetleaf*

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Propazine | 1.12 | - | - | - | - | 95 | |
| 2 | Propazine | 0.56 | | | | | 60 | |
| 3 | Propazine | 0.28 | | | | | 30 | |
| 4 | Simetryne | 1.12 | | | | | 70 | |
| 5 | Simetryne | 0.56 | | | | | 60 | |
| 6 | Simetryne | 0.28 | | | | | 15 | |
| 7 | | | EB-1 | 0.56 | | | 5 | |
| 8 | | | EB-1 | 0.28 | | | 0 | |
| 9 | Propazine | 1.12 | EB-1 | 0.56 | 2:1 | 95 | 80 | -16 |
| 10 | Propazine | 0.56 | EB-1 | 0.28 | 2:1 | 60 | 70 | 16 |
| 11 | Simetryne | 1.12 | EB-1 | 0.56 | 2:1 | 72 | 75 | 4 |
| 12 | Simetryne | 0.56 | EB-1 | 0.28 | 2:1 | 60 | 75 | 25 |

*Velvetleaf was 5 cm tall, 4 leaf stage, at time of treatment. Control of velvetleaf was determined 8 days following treatment.

TABLE 11

Control of Velvetleaf*

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Simazine | 2.24 | | | | | 5 | |
| 2 | | | EB-1 | 1.12 | | | 5 | |
| 3 | Simazine | 2.24 | EB-1 | 1.12 | 2:1 | 10 | 15 | 50 |

*Velvetleaf was 5 to 10 cm tall at time of treatment. Above evaluation was made 8 days following application.

TABLE 12

Control of Yellow Foxtail*

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Simazine | 2.24 | | | | | 0 | |
| 2 | | | EB-1 | 1.12 | | | 10 | |
| 3 | Simazine | 2.24 | EB-1 | 1.12 | 2:1 | 10 | 15 | 50 |

*Yellow foxtail was 7.6 cm, 3 leaf stage, at time of treatment. Above evaluation was made 8 days after treatment.

In a sixth series of experiments various substituted epoxy butane compounds were examined for enhanced atrazine activity when combined with atrazine. In the first run the activity of pyridine:-2,6-dichloro-4-(2-(2,2,2-trichloroethyl)oxiranyl (EB-2) on crabgrass (Digitaria spp.) and velvetleaf (Abutilon theophrasti) was determined both singularly and in combination with atrazine. The epoxy butane EB-1, (1,1,1-trichloro-3,4-epoxy-3,3,5-dichlorophenyl)-butane, was included for comparative purposes. Both epoxy butane compounds were formulated as emulsifiable concentrates as previously described (see pages 10-13).

All applications were made at 187 L/ha in 1.25% 411F crop oil concentrate to potted plants with soil surface shielded by the addition of vermiculite. The vermiculite was removed immediately following applications. All treatments were made to three pot replicates. Six days after treatment the injury to the weed species was assessed relative to controls and mean values recorded as shown in Table 13 for crabgrass, and Table 14 for velvetleaf.

TABLE 13

Control of Crabgrass* by Epoxy Butane Compounds and Atrazine Combinations

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Atrazine | 1.12 | | | | | 5 | |
| 2 | Atrazine | 0.56 | | | | | 0 | |
| 3 | | | EB-1 | 0.56 | | | 40 | |
| 4 | | | EB-1 | 0.28 | | | 35 | |
| 5 | | | EB-2 | 0.56 | | | 60 | |
| 6 | | | EB-2 | 0.28 | | | 45 | |
| 7 | Atrazine | 1.12 | EB-1 | 0.56 | 2:1 | 43 | 55 | 28 |
| 8 | Atrazine | 0.56 | EB-1 | 0.28 | 2:1 | 35 | 50 | 43 |
| 9 | Atrazine | 1.12 | EB-2 | 0.56 | 2:1 | 62 | 70 | 13 |
| 10 | Atrazine | 0.56 | EB-2 | 0.28 | 2:1 | 45 | 65 | 44 |

*Crabgrass was 12.7 cm tall at time of application.

Above evaluation was made 6 days after treatment.

EB-2 = pyridine:2,6-dichloro-4-(2-(2,2,2-trichloroethyl)oxiranyl.

TABLE 14

Control of Velvetleaf* by Epoxy Butane Compounds and Atrazine Combinations

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Atrazine | 1.12 | | | | | 85 | |
| 2 | Atrazine | 0.56 | | | | | 75 | |
| 3 | | | EB-1 | 0.56 | | | 20 | |
| 4 | | | EB-1 | 0.28 | | | 10 | |
| 5 | | | EB-2 | 0.56 | | | 15 | |
| 6 | | | EB-2 | 0.28 | | | 5 | |
| 7 | Atrazine | 1.12 | EB-1 | 0.56 | 2:1 | 88 | 90 | 2 |
| 8 | Atrazine | 0.56 | EB-1 | 0.28 | 2:1 | 78 | 80 | 3 |
| 9 | Atrazine | 1.12 | EB-2 | 0.56 | 2:1 | 87 | 90 | 3 |
| 10 | Atrazine | 0.56 | EB-2 | 0.28 | 2:1 | 76 | 85 | 12 |

*Velvetleaf was 5 cm tall at time of treatment. Above evaluation was made 6 days following application.

In another run, the enhanced triazine activity of oxirane:2-(3-chlorophenyl)-2-(2,2,2-trichloroethyl)- (EB-3), and oxirane:(2-phenyl-2-(2,2,2--trichloroethyl)- (EB-4) were evaluated. Both were formulated as emulsible concentrates as previously described and both were applied singularly and in combination with atrazine.

Test species in this second run also included crabgrass and velvetleaf, and compound EB-1 was included for comparative purposes. Each treatment was made to three pot replicates utilizing 1.25% 411F crop oil concentrate as the adjuvent. The results were averaged and evaluated for enhanced control as shown in Table 15 for crabgrass and 16 for velvetleaf.

TABLE 15

Crabgrass Control by EB-3, EB-4 Mixtures with Atrazine

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Atrazine | 1.12 | | | | | 60 | |
| 2 | Atrazine | 0.56 | | | | | 15 | |
| 3 | | | EB-3 | 0.56 | | | 20 | |
| 4 | | | EB-3 | 0.28 | | | 5 | |
| 5 | | | EB-4 | 0.56 | | | 25 | |
| 6 | | | EB-4 | 0.28 | | | 15 | |
| 7 | | | EB-1 | 0.56 | | | 55 | |
| 8 | | | EB-1 | 0.28 | | | 35 | |
| 9 | Atrazine | 1.12 | EB-3 | 0.56 | 2:1 | 68 | 80 | 18 |
| 10 | Atrazine | 0.56 | EB-3 | 0.28 | 2:1 | 19 | 55 | 189 |
| 11 | Atrazine | 1.12 | EB-4 | 0.56 | 2:1 | 70 | 80 | 14 |
| 12 | Atrazine | 0.56 | EB-4 | 0.28 | 2:1 | 28 | 65 | 132 |

TABLE 15 con't

Crabgrass Control by EB-3, EB-4
Mixtures with Atrazine

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 13 | Atrazine | 1.12 | EB-1 | 0.56 | 2:1 | 82 | 95 | 16 |
| 14 | Atrazine | 0.56 | EB-1 | 0.28 | 2:1 | 45 | 90 | 100 |

*Crabgrass was approximately 7.6 cm tall at time of treatment. Above evaluation was made 7 days after applications.

EB-3 = oxirane:2-(3-chlorophenyl)-2-(2,2,2-trichloroethyl)-

EB-4 = oxirane:2-phenyl-2-(2,2,2-trichloroethyl)-

TABLE 16

Velvetleaf Control with EB-3, EB-4
Mixtures with Atrazine

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Atrazine | 1.12 | | | | | 92 | |
| 2 | Atrazine | 0.56 | | | | | 90 | |
| 3 | | | EB-3 | 0.56 | | | 0 | |
| 4 | | | EB-4 | 0.56 | | | 0 | |
| 5 | | | EB-1 | 0.56 | | | 15 | |
| 6 | | | EB-1 | 0.28 | | | 5 | |
| 7 | Atrazine | 1.12 | EB-3 | 0.56 | 2:1 | 92 | 98 | 7 |
| 8 | Atrazine | 0.56 | EB-3 | 0.28 | 2:1 | 90 | 100 | 11 |
| 9 | Atrazine | 1.12 | EB-4 | 0.56 | 2:1 | 92 | 100 | 9 |
| 10 | Atrazine | 0.56 | EB-4 | 0.28 | 2:1 | 90 | 95 | 6 |
| 11 | Atrazine | 1.12 | EB-1 | 0.56 | 2:1 | 93 | 98 | 5 |
| 12 | Atrazine | 0.56 | EB-1 | 0.28 | 2:1 | 91 | 100 | 10 |

*Velvetleaf was 5 cm tall at time of treatment. Evaluation was made 7 days after application.

Example 6

Corn was planted in the field into a silt loam soil (3.5% organic matter) in the early spring. Approximately 3 weeks after planting when the corn was 10-12.7 cms tall (6 lf. stage) broadcast postemergent herbicide applications were made using conventional ground applications equipment. Giant foxtail represented the primary grass weed but some barnyard grass and green foxtail were present. Broadleaf weed presence was primarily from velvetleaf and annual morning glory with some occasional ivyleaf morning glory, pigweed, smart weed and horse nettle.

Atrazine was applied as Aatrex® 4L and EB-1 and EB-2 were applied as emulsifiable concentrates. All applications were made in the presence of 1.25% v/v 411F crop oil concentrate. Epoxy butane compounds EB-1 and EB-2 were applied both singularly and in combination with atrazine as previously described. Evaluations of grass and broadleaf weed control with the various herbicide rates and combinations (as well as corn injury) were assessed at various time intervals and those values obtained 16 days after treatment are reproduced in Table 17 for grass weeds, Table 18 for broadleaf weeds and Table 19 for corn injury.

TABLE 17

Grass Weed Control in the Field with Atrazine-Epoxy Butane Combination

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Atrazine | 1.8 | | | | | 35 | |
| 2 | | | EB-1 | 1.12 | | | 27 | |
| 3 | | | EB-1 | 0.56 | | | 10 | |
| 4 | | | EB-2 | 1.12 | | | 20 | |
| 5 | | | EB-2 | 0.56 | | | 0 | |
| 6 | Atrazine | 1.8 | EB-1 | 1.12 | 1.6:1 | 53 | 97 | 83 |
| 7 | Atrazine | 1.8 | EB-1 | 0.56 | 3.2:1 | 42 | 96 | 129 |
| 8 | Atrazine | 1.8 | EB-2 | 1.12 | 1.6:1 | 48 | 87 | 81 |
| 9 | Atrazine | 1.8 | EB-2 | 0.56 | 3.2:1 | 35 | 74 | 111 |

TABLE 18

Broadleaf Weed Control in the Field with Atrazine-Epoxy Butane Combination

| Run No. | Triazine Compound | Dosage, kg/ha | Epoxy Butane Compound | Dosage, kg/ha | Ratio of Triazine to Epoxy Butane | Expected Control In Percent | Actual Control In Percent | Percent Increase Over Expected Control |
|---|---|---|---|---|---|---|---|---|
| 1 | Atrazine | 1.8 | | | | | 97 | |
| 2 | | | EB-1 | 1.12 | | | 0 | |
| 3 | | | EB-2 | 1.12 | | | 0 | |
| 4 | Atrazine | 1.8 | EB-1 | 1.12 | 1.6:1 | 97 | 100 | 3 |
| 5 | Atrazine | 1.8 | EB-1 | 0.56 | 3.2:1 | 97 | 100 | 3 |
| 6 | Atrazine | 1.8 | EB-2 | 1.12 | 1.6:1 | 97 | 100 | 3 |
| 7 | Atrazine | 1.8 | EB-2 | 0.56 | 3.2:1 | 97 | 100 | 3 |

TABLE 19

Corn Injury With Atrazine-EB Combinations

| Run No. | Triazine Compound | Dosage, kg/ha | Epoxy Butane Compound | Dosage, kg/ha | Ratio of Triazine to Epoxy Butane | Expected Injury In Percent | Actual Injury In Percent | Percent Increase Over Expected Control |
|---|---|---|---|---|---|---|---|---|
| 1 | Atrazine | 1.8 | | | | | 0 | |
| 2 | | | EB-1 | 1.12 | | | 0 | |
| 3 | | | EB-2 | 1.12 | | | 0 | |
| 4 | Atrazine | 1.8 | EB-1 | 1.12 | 1.6:1 | 0 | 0 | 0 |
| 5 | Atrazine | 1.8 | EB-1 | 0.56 | 3.2:1 | 0 | 0 | 0 |
| 6 | Atrazine | 1.8 | EB-2 | 1.12 | 1.6:1 | 0 | 0 | 0 |
| 7 | Atrazine | 1.8 | EB-2 | 0.56 | 3.2:1 | 0 | 0 | 0 |

Example 7

Barnyard grass, johnsongrass, and yellow foxtail seeds were planted into a sandy loam soil in an agricultural region near Davis, California. The seeds received overhead irrigation and subsequent overhead irrigation as necessary. Atrazine (Aatrex® 4L), Cyanazine (Bladex® 80W), and the asymmetrical triazine metribuzin (Sencor®) were applied with a hand held compressed air sprayer calibrated to deliver 280.6 L/ha. Sun® 11E crop oil was added to all sprays to a final concentration of 1.7% v/v. The weeds were in the 4-5 leaf stage and 7.6-10 cms tall at time of treatment: all treatments were made in triplicate. The evaluation results obtained two weeks following application for the various triazines with or without the addition of the epoxy butane 1,1,1-trichloro-3,4-epoxy-3-(3,5--dichlorophenyl)butane, (EB-1) are set forth below in Table 20.

TABLE 20

Control of Seedling Grasses in the Field With Symmetrical and Asymmetrical Triazines and Epoxy Butane (EB-1) Combinations

| Triazine | kg/ha | Epoxy-Butane (kg/ha) | Ratio Triazine to Epoxy Butane | Control Rating BYG-2/ | YFT-2/ | JG-2/ | Ave. |
|---|---|---|---|---|---|---|---|
| Atrazine | 1.12 | - | | 40 | 40 | 0 | 27 |
| | 1.68 | - | | 57 | 47 | 0 | 35 |
| | 1.12 | 0.56 | 2:1 | 60 | 67 | 20 | 49 |
| | 1.68 | 0.56 | 3:1 | 83 | 70 | 23 | 59 |
| Cyanazine | 1.12 | - | | 30 | 57 | 10 | 32 |
| | 1.68 | - | | 50 | 70 | 3 | 41 |
| | 1.12 | 0.56 | 2:1 | 67 | 70 | 0 | 46 |
| | 1.68 | 0.56 | 3:1 | 77 | 83 | 0 | 53 |
| Metribuzin | 0.74 | - | | 73 | 75 | 20 | 56 |
| | 1.12 | - | | 75 | 85 | 30 | 63 |
| | 0.74 | 0.56 | 1.3:1 | 88 | 83 | 43 | 71 |
| | 1.12 | 0.56 | 2:1 | 95 | 96 | 67 | 86 |

-2/ BYG = Barnyardgrass
YFT = Yellow Foxtail
JG = Johnsongrass

The following examples 8, 9 and 10 present additional data relevant to the invention described herein. It will be noted that tested ratios of the triazine herbicide to the substituted oxirane herbicide fall largely outside the preferred range of 1.5 to 4 parts triazine to one part epoxy butane. There are no examples in these tests in the most preferred range of 2 to 3 parts triazine to 1 part substituted oxirane.

Examples 8, 9 and 10

In an additional series of runs illustrating the present composition and its use postemergently, duplicate plots were selected in a randomized design using standard precision procedures with 2 replicates of each plot, i.e., each trial. Three locations were selected representative of silt loam, clay and peat soil types respectively. The soils were infested with crab grass (Digitaria sanguinalis) as the main grassy weed and of the broadleaf weeds, willowweed (Polygonum persicaria), fathen (Chenopodium album), redroot pigweed (Amaranthus retroflexus) and sorrel (Rumex acetosa). The control was estimated on a 0 to 100 scale with 100 being complete control.

The commercial triazine product ACTAZINE 4A, a flowable concentrate containing a surfactant and 400 grams per liter of finely suspended atrazine was diluted to a tank mix and applied at the rate of 1.6 kilograms per hectare (kg/ha). One kilogram per hectare is equivalent to 2.2 lbs/2.47 acres or 0.89 lb/acre.

The substituted oxirane compound, 1,1,1-trichloro-3,4-epoxy-3-(3,5-dichlorophenyl)butane,. (EB-1) in

the form of an emulsifiable concentrate containing a surfactant and 480 grams per liter of the substituted oxirane was diluted to a tank mix in the form of an aqueous emulsion and applied to respective duplicated plots at the respective rates of 1, 1.5 and 2 kg/ha.

The substituted oxirane concentrate and the atrazine concentrate were also diluted and combined into respective aqueous tank mixes containing atrazine and substituted oxirane in weight ratios of 1.6:1 and 1.6:1.5. The tank mixes were applied to respective duplicated plots at the respective rates of (a) 1.6 kg/ha atrazine and 1 kg/ha substituted oxirane and (b) 1.6 kg/ha atrazine and 1.5 kg/ha substituted oxirane. In the silt loam plots weeds were not past the 2 leaf stage; in the clay plots the weeds were about at the 2 leaf stage, while in the peat plots, weeds were in various stages of growth up to the 4 leaf stage. In each case, the corn had also germinated.

The results were evaluated 4 and 6 weeks after treatment of the silt loam and clay plots, and after each of 6 and 8 weeks in the case of the peat plots.

The results of these runs are summarized in the following Tables 21, 22, 23, 24 and 25. In each case, crop selectivity was excellent as no crop damage to corn was observed.

TABLE 21

Postemergent Control of Grassy Weeds in Corn - Silt Loam Plot

| Run No. | Active Component | Dosage kg/ha | Ratio of Chemicals A:EB-1 | Evaluation After 4 Weeks: Expected Control Percent | Evaluation After 4 Weeks: Actual Control Percent | Evaluation After 4 Weeks: Percent Increase Over Expected |
|---|---|---|---|---|---|---|
| 1 | EB-1 | 1 | - | - | 81 | - |
| 2 | EB-1 | 1.5 | - | - | 90 | - |
| 3 | A | 1.6 | - | - | 40 | - |
| 4 | A + EB-1 | 1.6 + 1 | 1.6:1 | 89 | 84 | -6 |
| 5 | A + EB-1 | 1.6 + 1.5 | 1.1:1 | 94 | 98 | 4 |

TABLE 21 Con'd

Postemergent Control of Grassy Weeds in Corn - Silt Loam Plot

| Run No. | Active Component | Dosage kg/ha | Ratio of Chemicals A:EB-1 | Evaluation After 6 Weeks: Expected Control Percent | Actual Control Percent | Percent Increase Over Expected |
|---|---|---|---|---|---|---|
| 1 | EB-1 | 1 | - | - | 80 | - |
| 2 | EB-1 | 1.5 | - | - | 90 | - |
| 3 | A | 1.6 | - | - | 20 | - |
| 4 | A + EB-1 | 1.6 + 1 | 1.6:1 | 84 | 85 | 1 |
| 5 | A + EB-1 | 1.6 + 1.5 | 1.1:1 | 92 | 95 | 3 |

A = atrazine.

Expected control and percent increase over expected calculated as shown in Table 1.

TABLE 22

Postemergent Control of Broadleaf Weeds in Corn - Silt Loam Plot

| Run No. | Active Component | Dosage kg/ha | Ratio of Chemicals A:EB-1 | Evaluation After 4 Weeks: Expected Control Percent | Actual Control Percent | Percent Increase Over Expected |
|---|---|---|---|---|---|---|
| 1 | EB-1 | 1 | - | - | 40 | - |
| 2 | EB-1 | 1.5 | - | - | 58 | - |
| 3 | A | 1.6 | - | - | 93 | - |
| 4 | A + EB-1 | 1.6 + 1 | 1.6:1 | 96 | 97 | 1 |
| 5 | A + EB-1 | 1.6 + 1.5 | 1.1:1 | 97 | 100 | 3 |

TABLE 22 Con'd

Postemergent Control of Broadleaf Weeds in Corn - Silt Loam Plot

| Run No. | Active Component | Dosage kg/ha | Ratio of Chemicals A:EB-1 | Evaluation After 6 Weeks: Expected Control Percent | Evaluation After 6 Weeks: Actual Control Percent | Evaluation After 6 Weeks: Percent Increase Over Expected |
|---|---|---|---|---|---|---|
| 1 | EB-1 | 1 | - | - | 10 | - |
| 2 | EB-1 | 1.5 | - | - | 20 | - |
| 3 | A | 1.6 | - | - | 90 | - |
| 4 | A + EB-1 | 1.6 + 1 | 1.6:1 | 91 | 90 | -1 |
| 5 | A + EB-1 | 1.6 + 1.5 | 1.1:1 | 92 | 93 | 1 |

A = atrazine.

Expected control and percent increase over expected calculated as shown in Table 1.

TABLE 23

Postemergent Control of Grassy Weeds in Corn - Clay Plot

| Run No. | Active Component | Dosage kg/ha | Ratio of Chemicals A:EB-1 | Evaluation After 4 Weeks: Expected Control Percent | Actual Control Percent | Percent Increase Over Expected |
|---|---|---|---|---|---|---|
| 1 | EB-1 | 1 | - | - | 38 | - |
| 2 | EB-1 | 1.5 | - | - | 55 | - |
| 3 | A | 1.6 | - | - | 15 | - |
| 4 | A + EB-1 | 1.6 + 1 | 1.6:1 | 47 | 53 | 13 |

TABLE 23 Con'd

Postemergent Control of Grassy Weeds in Corn - Clay Plot

| | | | | Evaluation After 6 Weeks | | |
|---|---|---|---|---|---|---|
| Run No. | Active Component | Dosage kg/ha | Ratio of Chemicals A:EB-1 | Expected Control Percent | Actual Control Percent | Percent Increase Over Expected |
| 1 | EB-1 | 1 | - | - | 10 | - |
| 2 | EB-1 | 1.5 | - | - | 23 | - |
| 3 | A | 1.6 | - | - | 5 | - |
| 4 | A + EB-1 | 1.6 + 1 | 1.6:1 | 15 | 30 | 100 |

A = atrazine.

Expected control and percent increase over expected calculated as shown in Table 1.

TABLE 24

Postemergent Control of Broadleaf Weeds in Corn - Clay Plot

| Run No. | Active Component | Dosage kg/ha | Ratio of Chemicals A:EB-1 | Evaluation After 4 Weeks: Expected Control Percent | Actual Control Percent | Percent Increase Over Expected |
|---|---|---|---|---|---|---|
| 1 | EB-1 | 1 | - | - | 10 | - |
| 2 | EB-1 | 1.5 | - | - | 28 | - |
| 3 | A | 1.6 | - | - | 65 | - |
| 4 | A + EB-1 | 1.6 + 1 | 1.6:1 | 69 | 78 | 13 |

TABLE 24 Con'd

Postemergent Control of Broadleaf Weeds in Corn - Clay Plot

| Run No. | Active Component | Dosage kg/ha | Ratio of Chemicals A:EB-1 | Evaluation After 6 Weeks: Expected Control Percent | Evaluation After 6 Weeks: Actual Control Percent | Evaluation After 6 Weeks: Percent Increase Over Expected |
|---|---|---|---|---|---|---|
| 1 | EB-1 | 1 | - | - | 0 | - |
| 2 | EB-1 | 1.5 | - | - | 5 | - |
| 3 | A | 1.6 | - | - | 35 | - |
| 4 | A + EB-1 | 1.6 + 1 | 1.6:1 | 35 | 58 | 66 |

A = atrazine.

Expected control and percent increase over expected calculated as shown in Table 1.

TABLE 25

Postemergent Control of Grassy Weeds in Corn - Peat Plot

| Run No. | Active Component | Dosage kg/ha | Ratio of Chemicals A:EB-1 | Evaluation After 6 Weeks: Expected Control Percent | Actual Control Percent | Percent Increase Over Expected |
|---|---|---|---|---|---|---|
| 1 | EB-1 | 1 | - | - | 68 | - |
| 2 | EB-1 | 1.5 | - | - | 68 | - |
| 3 | A | 1.6 | - | - | 0 | - |
| 4 | A + EB-1 | 1.6 + 1 | 1.6:1 | 68 | 75 | 10 |
| 5 | A + EB-1 | 1.6 + 1.5 | 1.1:1 | 68 | 85 | 25 |

TABLE 25 Con'd

Postemergent Control of Grassy Weeds in Corn - Peat Plot

| | | | | Evaluation After 8 Weeks | | |
|---|---|---|---|---|---|---|
| Run No. | Active Component | Dosage kg/ha | Ratio of Chemicals A:EB-1 | Expected Control Percent | Actual Control Percent | Percent Increase Over Expected |
| 1 | EB-1 | 1 | - | - | 43 | - |
| 2 | EB-1 | 1.5 | - | - | 40 | - |
| 3 | A | 1.6 | - | - | 10 | - |
| 4 | A + EB-1 | 1.6 + 1 | 1.6:1 | 49 | 48 | -2 |
| 5 | A + EB-1 | 1.6 + 1.5 | 1.1:1 | 46 | 75 | 63 |

A = atrazine.

Expected control and percent increase over expected calculated as shown in Table 1.

Example 11

The treatment protocol of Examples 8-10 was followed in two different locations, each with a fairly high organic content. The principal grassy weeds infesting the plots were summer grass (Digitarea sanguinalis) and barnyard grass (Echinochloa crus-galli). In this case the weeds were sprayed with the various tank mixes at the 2 to 5 leaf stage in a first plot and at the 3 to 6 leaf stage in a second plot, in each case, the corn having germinated. At the second plot, the atrazine was also applied alone at 1 kg/ha alone and in combination with the epoxy butane compound at various rates of the latter. In each case, the grassy weed control and crop injury was assessed 14 days after treatment and again at least 6 weeks after treatment. The weed control results are summarized in Table 26. No crop injury was observed.

TABLE 26

Postemergent Control of Grassy Weeds in Corn

| Run No. | Active Component | Dosage kg/ha | Ratio of Chemicals A:EB-1 | Evaluation After 14 Days: Plot 1 Expected Control Percent | Plot 1 Actual Control Percent | Plot 1 Percent Increase Over Expected | Plot 2 Expected Control Percent | Plot 2 Actual Control Percent | Plot 2 Percent Increase Over Expected |
|---|---|---|---|---|---|---|---|---|---|
| 1 | EB-1 | 1 | - | - | 58 | - | - | 51 | - |
| 2 | EB-1 | 1.5 | - | - | 60 | - | - | 57 | - |
| 3 | A | 1 | - | - | NR | - | - | 18 | - |
| 4 | A | 1.6 | - | - | 20 | - | - | 21 | - |
| 5 | A + EB-1 | 1.6 + 1 | 1.6:1 | 66 | 83 | 26 | 61 | 67 | 10 |
| 6 | A + EB-1 | 1.6 + 1.5 | 1.1:1 | 68 | 85 | 25 | 66 | 71 | 8 |
| 7 | A + EB-1 | 1 + 1 | 1:1 | - | NR | - | 60 | 67 | 12 |

TABLE 26 con't

Postemergent Control of Grassy Weeds in Corn

| Run No. | Active Component | Dosage kg/ha | Ratio of Chemicals A:EB-1 | Evaluation After 6 Weeks: Plot 1 Expected Control Percent | Plot 1 Actual Control Percent | Plot 1 Percent Increase Over Expected | Plot 2 Expected Control Percent | Plot 2 Actual Control Percent | Plot 2 Percent Increase Over Expected |
|---|---|---|---|---|---|---|---|---|---|
| 1 | EB-1 | 1 | - | - | 50 | - | - | 97 | - |
| 2 | EB-1 | 1.5 | - | - | 75 | - | - | 97 | - |
| 3 | A | 1 | - | - | NR | - | - | 100 | - |
| 4 | A | 1.6 | - | - | 35 | - | - | 93 | - |
| 5 | A + EB-1 | 1.6 + 1 | 1.6:1 | 68 | 85 | 25 | 100 | 100 | 0 |
| 6 | A + EB-1 | 1.6 + 1.5 | 1.1:1 | 84 | 90 | 7 | 100 | 100 | 0 |
| 7 | A + EB-1 | 1 + 1 | 1.0:1 | - | NR | - | 100 | 98 | -2 |

A = atrazine; NR = not run.

Expected control and percent increase over expected calculated as shown in Table 1.

Example 12

An aqueous tank mix was prepared from a commercial flowable suspension of atrazine containing about 0.48 kg/L active ingredient, adding a crop oil concentrate and water. The concentration of active ingredient in the tank mix was about 0.8 percent by weight.

A second tank mix was prepared using the same flowable suspension of atrazine, an emulsion concentrate of the epoxy butane compound, 1,1,1-trichloro-3,4-epoxy-3-(3,5-dichlorophenyl)butane, and a crop oil concentrate in the proportions of 3.9 parts by weight atrazine per part of epoxy butane compound and 0.9463 L of crop oil concentrate for each 0.68 kg of atrazine. The concentrations of atrazine and epoxy butane compound, respectively, in the tank mix were 0.6 percent and 0.16 percent by weight.

The respective tank mixes were applied to respective field plots 22 days after each was seeded to corn when grassy weeds, particularly giant foxtail seedlings, were about 3.8 cms tall and the corn was 10 to 12.7 cms tall. Application was made by spraying the tank mixes at the rate of 280 L/ha, in the requisite amount to provide on a first plot 2.24 kgs/ha and 2.34 L/ha crop oil concentrate, and on a second plot 1.68 kg/ha atrazine, 0.42 kg/ha epoxy butane compound and 2.34 L/ha crop oil concentrate.

The respective tank mixes were also applied to respective third and fourth field plots seeded to

corn 35 days after planting when seedling weeds such as giant foxtail were about 6.35 cms tall and the corn 12.7 to 15.2 cms tall.

These field tests were evaluated 27 days after the early post treatment and 24 days after the post treatments. No damage to the corn was noted and the following tabulated results on giant foxtail and velvet leaf populations were observed:

TABLE 27

| Tank Mix Ingredients | Rate Per ha | Time of Treatment | % Control Giant Foxtail | % Control Velvet Leaf |
|---|---|---|---|---|
| 1. atrazine<br>crop oil | 2.24 kg<br>2.34 L | 22 days post | 54 | 99 |
| 2. atrazine<br>epoxy butane<br>crop oil | 1.68 kg<br>.42 kg<br>2.34 L | 22 days post | 96 | 98 |
| | | 25 days post | 91 | 100 |

Example 13

Five respective aqueous tank mixes were made up using, variously, a commercial suspension concentrate of atrazine, Bladex® 80W brand of wettable powder containing 80% by weight cyanazine, an emulsion concentrate of the epoxy butane compound, 1,1,1-trichloro-3,4-epoxy-3-(3,5-dichlorophenyl)butane, and a crop oil concentrate.

Two of the tank mixes contained, respectively, atrazine and cyanazine, while three other tank mixes each contained epoxy butane compound, and, respectively, in addition, atrazine and crop oil, and atrazine and cyanazine.

Respective field plots seeded to corn were treated with respective tank mixes postemergently 13 days afterthe corn was planted, and thus prior to indigenous wild proso millet reaching the 8 leaf stage. The tank mixes were sprayed upon the plots at the requisite rate in the range of 187 to 280 L/ha to provide the dosages set forth in Table 28. The extent of control of proso millet was noted 16 days after treatment and again 50 days after treatment. The treatments and results are tabulated in Table 28. No crop injury was noted.

TABLE 28

| Tank Mix Ingredients | Rate Per Hectare | % Control, Wild Proso Millet 2 Weeks After Treatment | 7 Weeks After Treatment |
|---|---|---|---|
| 1. atrazine | 2.24 kg | 32 | 27 |
| 2. atrazine<br>epoxy butane | 2.24 kg<br>0.56 kg | 74 | 48 |
| 3. atrazine<br>epoxy butane<br>crop oil | 2.24 kg<br>0.56 kg<br>1.17 L | 73 | 45 |
| 4. cyanazine | 2.24 kg | 56 | 42 |
| 5. cyanazine<br>epoxy butane | 2.24 kg<br>0.56 kg | 84 | 82 |

Example 14

Aqueous tank mixes were made up containing atrazine and crop oil concentrate, and, atrazine, epoxy butane compound and crop oil concentrate. The atrazine was utilized in the form of a commercial flowable concentrate, the epoxy butane compound, 1,1,1-trichloro--3,4-epoxy-3-(3,4-dichlorophenyl)butane, was utilized in the form of an emulsion concentrate, and the crop oil concentrate was Sun® 11E brand.

Respective field plots seeded to corn were treated with spray applications of respective tank mixes when the indigenous giant foxtail population was about 7.6 to 10 cms tall. The control of gian foxtail was evaluated visually 7 days and again 29 days after treatment. The results and dosage rates are tabulated in Table 29.

TABLE 29

Control of Giant Foxtail

| Tank Mix Ingredients | Rate Per Hectare | 7 Days After Treatment | 29 Days After Treatment |
|---|---|---|---|
| 1. atrazine | 1.68 kg | 92 | 93 |
| crop oil | 2.34 L | | |
| 2. atrazine | 1.68 kg | 100 | 98 |
| epoxy butane | 0.28 kg | | |
| crop oil | 1.17 L | | |

On repeating the second test but replacing one-half of the atrazine with an equal amount of cyanazine whereby the dosage rate per hectare was 0.84 kg atrazine and 0.84 kg cyanazine the same excellent control was observed. No crop injury was noted.

CLAIMS

1. A herbicidal composition for use in postemergence agricultural field operations which comprises in admixture an agriculturally acceptable carrier and an effective amount in the range of, by weight, from one to eight parts of at least one herbicidal triazine and a 1,1,1-trichloro-3,4-epoxy butane compound having in the 3-position a phenyl, substituted phenyl or substituted 4-pyridinyl group, the ring substituents on the substituted phenyl group being in one or more of the 3-, 4- and 5-positions and each being a hydrogen, chlorine, bromine or fluorine atom, or a trifluoromethyl, nitro, $C_{1-3}$ alkyl or $C_{1-3}$ alkoxy group, and the ring substituents on the substituted 4-pyridinyl group being 1 or 2 in number and each being a chlorine, bromine or fluorine atom, or a trifluoromethyl, nitro, $C_{1-3}$ alkyl or $C_{1-3}$ alkoxy group.

2. A composition as claimed in claim 1 wherein the herbicidal triazine is atrazine, cyanazine or a mixture thereof.

3. A composition as claimed in claim 1 or claim 2 wherein the epoxy butane has a substituted phenyl group at the 3-position and the ring substituent or

substituents are 3- or 3,5 fluorine, chlorine, bromine, trifluoromethyl or methyl.

4. A composition as claimed in any one of claims 1 to 3 which contains 1 to 4 parts of herbicidal triazine per part of epoxy butane.

5. A composition as claimed in claim 4 which contains 2 to 3 parts of herbicidal triazine per part of epoxy butane.

6. A method of controlling grassy weeds which comprises applying postemergently to the weeds a herbicidally effective amount of a composition as claimed in any one of the preceding claims in the range of 0.56 to 5 kgs per hectare based on the weight of active ingredients in the composition and utilizing sufficient composition to provide at least 0.28 kgs per hectare of epoxy butane compound.

7. A method as claimed in claim 6 wherein sufficient of the said composition is utilized to provide at least 0.56 kg/ha epoxy butane compound.

8. A method as claimed in claim 6 or claim 7 wherein the composition is applied in an effective amount in the range of 1.12 to 2.36 kgs per hectare.

9. A method as clained in any of claims 6 to 8 which is carried out on crops of corn or sorghum.

0081351

European Patent Office

# EUROPEAN SEARCH REPORT

Application number

EP 82 30 6445

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A,D | US-A-3 719 465 (T.M.OZRETICH)<br>--- | 1 | A 01 N 43/70<br>A 01 N 43/64<br>A 01 N 43/40<br>C 07 D 405/04 //<br>(A 01 N 43/70<br>A 01 N 43/40<br>A 01 N 43/20 )<br>(A 01 N 43/64<br>A 01 N 43/40<br>A 01 N 43/20 ) |
| A | US-A-3 930 835 (T.M.OZRETICH)<br>--- | 1 | |
| A,D | US-A-4 211 549 (L.D.MARKLEY)<br>----- | 1 | |
| | | | TECHNICAL FIELDS SEARCHED (Int. Cl. 3) |
| | | | A 01 N |

The present search report has been drawn up for all claims

| Place of search | Date of completion of the search | Examiner |
|---|---|---|
| THE HAGUE | 17-03-1983 | DECORTE D. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82